# EUROPEAN PATENT SPECIFICATION

(11) **EP 4 123 411 B1**
(45) Date of publication and mention of the grant of the patent: **11.09.2024**
(21) Application number: 22181311.6
(22) Date of filing: 27.06.2022
(51) Int. Cl.: G05D 23/27

(54) **COMPACT OPTICAL HIGH-SPEED SYSTEM FOR NUCLEIC ACID AMPLIFICATION AND DETECTION**
KOMPAKTES OPTISCHES HOCHGESCHWINDIGKEITSSYSTEM ZUR AMPLIFIKATION UND DETEKTION VON NUKLEINSÄURE
SYSTÈME OPTIQUE COMPACT À HAUTE VITESSE POUR L'AMPLIFICATION ET LA DÉTECTION D'ACIDES NUCLÉIQUES

(30) Priority: 29.06.2021 US 202163216421 P; 23.01.2022 US 202263302108 P; 14.03.2022 US 202217694581
(43) Date of publication of application: 25.01.2023
(73) Proprietor: Samsung Electronics Co., Ltd., Gyeonggi-do, 16677 (KR)
(72) Inventor: NARASIMHAN, Vinayak, San Jose, CA, 95134 (US); WANG, Yibing Michelle, San Jose, CA, 95134 (US); SIDDIQUE, Radwanul Hasan, San Jose, CA, 95134 (US)
(74) Representative: Kuhnen & Wacker Patent- und Rechtsanwaltsbüro PartG mbB

(56) References cited:
- EP-A1- 0 640 828
- WO-A1-2014/100725
- WO-A1-2017/127570

## Description

### FIELD

Aspects of the invention relate to the field of nucleic acid detection systems and methods of using the same.

### BACKGROUND

Various nucleic acid (NA) amplification strategies exist in the market, including but not limited to, polymerase chain reaction (PCR), loop-mediated isothermal amplification (LAMP), nucleic acid sequence based amplification (NASBA), strand displacement amplification (SDA), multiple displacement amplification (MDA), rolling circle amplification (RCA), ligase chain reaction (LCR), helicase dependent amplification (HDA), ramification amplification method (RAM), recombinase polymerase reaction (RPA), whole genome amplification (WGA), etc. Amplification occurs by creating copies of the target nucleic acid (DNA/RNA) cyclically, and generally involves heating, monitoring of temperature, and optical signals, including but not limited to, fluorescence, color, turbidity, etc. Generally, the presence or absence of optical signals beyond a certain threshold is used to determine the corresponding presence or absence of the target sequence.

In the amplification methods of the related art, which are not isothermal, thermocycling is often performed using bulky tabletop equipment and onboard electronics.

The above information disclosed in this Background section is only for enhancement of understanding of the background of the invention, and therefore it may contain information that does not form the prior art.

WO 2017/127570 A1 discloses: A method of performing one or more nucleic acid amplification reactions comprising at least one heating cycle, said method comprising receiving one or more samples at one or more reaction zones, generating heat at said one or more reaction zones by illuminating a heat generation layer in thermal communication with said one or more reaction zones, and performing nucleic acid amplification reactions on said one or more samples.

### SUMMARY

Aspects of embodiments of the present invention are directed toward a compact optical system for NA amplification that is capable of greatly reducing assay time by monitoring NA amplification in real-time with high sensitivity. The optical system does not require any dedicated equipment for thermal cycling or isothermal amplification, thereby reducing size and cost of the overall system.

According to some exemplary embodiments of the present invention, there is provided a system for nucleic acid (NA) amplification, the system including: a light source configured to emit a first excitation light based on a control signal; a reaction chamber configured to house a solution including a plurality of first nucleic acids (NAs), the plurality of first NAs being configured to amplify in response to the first excitation light, the solution being configured to emit a second light in response to heating by the first excitation light and to emit a third light in response to amplification of the plurality of first NAs; a detector configured to detect the second and third lights and to generate a temperature signal corresponding to the second light and a first fluorescence signal corresponding to the third light; and a lens module configured to focus the second and third lights onto the detector. The system further includes: a mirror configured to pass-through the first excitation light and to direct the second and third lights toward the lens module.

In some embodiments, the system further includes: a controller configured to generate the control signal to pulse the first excitation light based on the temperature signal, the control signal having a variable pulse width and being based on the temperature signal and a desired temperature of the solution, wherein the controller is further configured to determine presence of the plurality of first NAs in the solution based on the first fluorescence signal.

In some embodiments, the light source includes a blue light emitting diode (LED), the first excitation light has a blue range of wavelengths, the second light is in a long wavelength infrared (LWIR) range, and the third light has an orange range of wavelengths.

In some embodiments, the detector includes: a first pixel array configured to detect the second light and to generate the temperature signal corresponding to the second light; and a second pixel array configured to detect the third light and to generate the first fluorescence signal corresponding to the third light.

In some embodiments, the temperature signal is an average of intensities of light detected by each one of pixels across the first pixel array, and the first fluorescence signal is an average of intensities of light detected by each one of pixels across the second pixel array.

In some embodiments, the second pixel array includes a cooled infrared photodetector or an uncooled photodetector, and the second pixel array includes at least one of an avalanche photodiode (APD), a quanta image sensor (QIS), and a single-photon avalanche diode (SPAD).

In some embodiments, the lens module includes: a first metalens; a second metalens; and a third metalens, wherein the first metalens is configured to focus the second light onto the second metalens and to focus the third light onto the third metalens, and wherein the second metalens is configured to focus the second light onto the first pixel array, and the third metalens is configured to focus the third light onto the second pixel array.

In some embodiments, the second and third metalenses are offset from one another in a direction crossing an optical axis of the first metalens.

In some embodiments, the second and third metalenses are aligned with one another along an optical axis of the first metalens.

In some embodiments, the plurality of first NAs include at least one of first RNAs and first DNAs, and the solution includes fluorophores that combine with the plurality of first NAs and fluoresce in response to receiving the first excitation light.

In some embodiments, the light source includes a green LED configured to generate a second excitation light in response to the control signal, and the solution further includes a plurality of second NAs being configured to amplify in response to the second excitation light, the solution being configured to emit a fourth light in response to amplification of the plurality of second NAs.

In some embodiments, the system further includes: a third pixel array configured to detect the fourth light and to generate a second fluorescence signal corresponding to the third light; and a controller / the controller configured to determine a concentration of the plurality of second NAs in the solution based on the second fluorescence signal.

In some embodiments, the reaction chamber includes a plurality of wells, one or more of the plurality of wells including the plurality of first NAs, the second light includes one or more fluorescent lights corresponding to the one or more of the plurality of wells, and the first fluorescence signal includes a two-dimensional image contrasting the one or more of the plurality of wells from other wells of the plurality of wells.

In some embodiments, the system further includes: a controller / the controller configured to determine a concentration of the plurality of first NAs in the plurality of wells based on the two-dimensional image.

### BRIEF DESCRIPTION OF THE DRAWINGS

The above and other features and aspects of the invention will be made more apparent by the following detailed description of exemplary embodiments thereof with reference to the attached drawings, in which:
FIG. 1 is a schematic diagram of a compact optical system for nucleic acid (NA) amplification, according to some exemplary embodiments of the present invention.
FIGS. 2A-2B are schematic diagrams of compact optical systems that are capable of amplification and assay of two different types of NAs in the reaction chamber, according to some exemplary embodiments of the present invention.
FIG. 3 is a schematic diagram of a compact optical system capable of rapid end-point amplification and assay, according to some exemplary embodiments of the present invention.
FIG. 4 is a schematic diagram of a compact optical system capable of rapid end-point amplification and assay of more than one type of NA, according to some exemplary embodiments of the present invention.
FIGS. 5A-5C are schematic diagrams of the lens module and detector utilizing different focusing lens and pixel array arrangements, according to some embodiments of the present disclosure.
FIG. 6 is a flow diagram illustrating the process of optical NA amplification, according to some embodiments of the present disclosure.

### DETAILED DESCRIPTION

The attached drawings for illustrating exemplary embodiments of the invention are referred to in order to provide a sufficient understanding of the invention, the merits thereof, and the objectives accomplished by the implementation of the invention. The invention may, however, be embodied in many different forms and should not be construed as being limited to the exemplary embodiments set forth herein; rather, these exemplary embodiments are provided so that this disclosure will be thorough and complete, and will fully convey the concept of the invention to those skilled in the art.

Hereinafter, the invention will be described in detail by explaining exemplary embodiments of the invention with reference to the attached drawings. In the drawings, like reference numerals are used throughout the figures to reference like features and components.

According to some embodiments of the present disclosure, the optical nucleic acid (NA) amplification and assay system (henceforth referred to as the "optical system") utilizes the same excitation light to both excite the target NAs for isothermal amplification and also to heat the solution containing the target NAs for thermocycling. The optical system monitors the thermal radiation from the solution to determine its temperature, and adjusts the temperature by appropriately pulsing the excitation light. This enables accurate thermocycling of the solution without the use of bulky and costly electronic heating systems. The optical system is also capable of accurately tracking fluorescence of amplified NAs as a function of time by using a highly sensitive detector, which enables reduced thermal cycling and assay time. The optical system may be used with quantitative NA amplification schemes that provide either absolute or relative quantification using standards, references, endogenous controls, and exogenous controls.

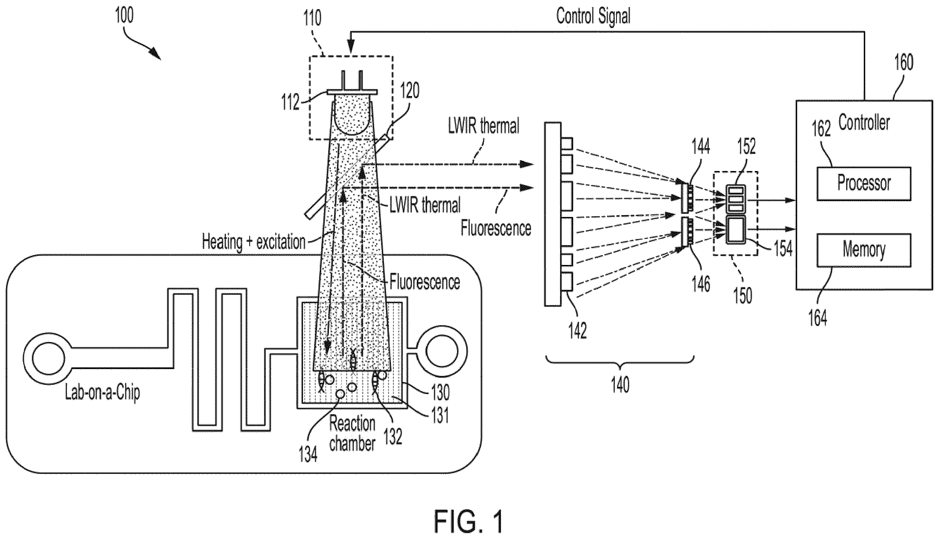

FIG. 1 is a schematic diagram of a compact optical system 100 for nucleic acid (NA) amplification, according to some exemplary embodiments of the present invention.

In some embodiments, the optical system 100 includes a light source 110, a mirror 120, a reaction chamber 130, a lens module 140, a detector 150, and a controller 160.

The light source 110 emits a first excitation light toward the reaction chamber 130 based on a control signal from the controller 160. In some examples, the light source 110 includes a blue light emitting diode (LED) 112 that emits light in the wavelength range of about 450 nm to about 495 nm. In some embodiments, the light source 110 may be pulsed and have a variable frequency or variable pulse width as determined by the control signal. The reaction chamber 104 is configured to house a solution 131 that includes a plurality of first nucleic acids (NAs) 132 and a plurality of first fluorophores (e.g., first fluorescent molecules) 134, which bind to the first NAs and are sensitive to the first excitation light from the light source 110.

The excitation light has a frequency that is also capable of heating the solution 131. Once heated, the solution 131 emits a second light having a wavelength in the long-wavelength infrared range (e.g., about 8 µm to about 12 µm), which is directed toward the lens module (e.g., lens assembly) 140 by the mirror 120 and detected by the detector 150. The intensity of the second light represents the temperature of the solution 131.

According to some examples, the first NAs 132 may be target deoxyribonucleic acid (DNA) or ribonucleic acid (RNA) strands, which are sensitive to and are excited by the excitation light and undergo thermocycling or isothermal amplification in response to incidence of the first excitation light. The amplification of the first NAs may involve denaturing, annealing, and extension processes. As the first NAs amplify (e.g., grow in number), the first fluorophores 134 in the solution 131 bind to the first NAs and greatly increase their fluoresce, which results in the solution 131 emitting a third light (e.g., a fluorescence light) in response to the excitation and amplification of the first NAs. Because the fluorescence of the first fluorophores 134 greatly increases as they bind to the first NAs, the intensity of the third light noticeably increases as the first NAs 132 are excited and undergo amplification. Therefore, the intensity of the third light, which may have a wavelength in the range of visible to near-infrared (e.g., between about 400 nm to about 800 nm), represents the level of amplification of the first NAs 132. In some examples, the third light may be a green light having a wavelength in range of about 490 nm to about 530 nm. In some examples, the first fluorophores 134 may include fluorescein, rhodamine, cyanine, BODIPY-FL, 7-nitrobenz-2-oxa-1, 3-diazole-4-yl, naphthalimide (lucifer yellow), acridine orange, and/or the like.

The third light that is emitted from the solution 131 is directed toward the lens module 140 by the mirror 120 and is then detected by the detector 150.

The mirror 120 is positioned between the light source 110 and the reaction chamber 130 along the light path of the first light, and is angled with respect to the first light. In some examples, the mirror 120 includes a dichroic mirror (e.g., a dual-band mirror) that allows the first light from the light source 110, which may have a blue color, to pass through and reach the reaction chamber 130, and reflect other colors, i.e., the second and third lights. In some examples, the dichroic mirror includes alternating layers of optical coatings with different refractive indices that are stacked upon a glass substrate.

According to some embodiments, the lens module 140 includes a first metalens 142, a second metalens 144, and a third metalens 146. The first metalens 142 receives the second and third lights that are redirected by the mirror 120 and focuses the second light onto the second metalens 144 and focuses the third light onto the third metalens 146. The second metalens 144 in turn focuses the second light onto a first pixel array 152 of the detector 150, and the third metalens 146 focuses the third light onto the third pixel array 156 of the detector 150. Each of the metalenses 142-146 may be a flat surface lens that uses nanostructures to focus light. In some examples, the first metalens 142 may have a size in the range of about 100s of µm to several centimeters (e.g., when working as a global lens) and the second and third metalenses 144 and 146 may have a size in the range of several micrometers to several millimeters (e.g., when working as micro or macro lenses). Thus, by using flat, compact metalenses instead of the more commonly-used bulky, curved lenses, the optical system 100 can achieve a smaller size than the systems of the related art.

While FIG.1 illustrates an embodiment in which the lens module 140 includes three metalenses, embodiments of the present disclosure are not limited thereto. For example, a single metalens may be used to focus the second and third lights onto the first and second pixel arrays, respectively.

In some embodiments, the first pixel array 152 of the detector 150 detects the second light and generates a corresponding electrical signal (also referred to as a temperature signal) to be used by the controller 160. The generated signal may correspond to (e.g., be equal or proportional to) the average pixel value across the first pixel array 152. As the intensity of the second light is indicative of the temperature of the solution 131 in the reaction chamber 130 and because each pixel value corresponds to the intensity of the second light at that pixel, the signal generated by the first pixel array 152 corresponds to the solution temperature.

According to some embodiments, the controller 160 utilizes the temperature signal to monitor the actual temperature of the solution 131 in real-time. As the controller 160 can also affect the temperature of the solution 131 through adjusting the frequency or the pulse-width of the pulsed first excitation light (via the control signal), optically measuring the solution temperature in real-time enables the controller 160 to precisely control and adjust the temperature of the solution 131. As such, in some embodiments, the controller 160 is capable of subjecting the solution 131 to thermocycles that facilitate the amplification of the first NAs. As an example, in one amplification cycle, the controller 160 may gradually increase the solution temperature from 55 °C to 90 °C, then decrease it to 75 °C, and finally reduce it down to 55 °C. The controller 160 may initiate many amplification cycles in order to amplify the first NAs to a point where the presence and/or concentration of the first NAs can be detected/measured.

In some embodiments, the second pixel array 154 detects the third light (e.g., the fluorescence light) and generates a first fluorescence signal corresponding to the third light. The fluorescence signal may be an average of intensities of light detected by each one of the pixels across the second pixel array 154 and is used by the controller 160 to determine the presence and/or the starting concentration of the first NAs 132 in the solution 131.

In some embodiments, the controller 160 maintains a count of the thermocycles as it monitors the fluorescence of the solution 131. Once the fluorescence signal reaches a detection threshold value, the controller 160 may calculate the starting concentration of the first NAs 132 by utilizing a table that maps number of thermocycles to achieve the threshold value of fluorescence to known initial concentrations of the first NAs 132. The table may be stored at the memory 164. When a count of thermocycles in a particular assay process does not match the count values in the table, the controller 160 may interpolate between nearest tabulated count values and their corresponding starting concentrations to arrive at an estimate of initial concentration of the first NAs 132 in the reaction chamber 130 prior to excitation and amplification.

In some embodiments, the detector 150, which includes a plurality of pixel arrays, provides high detection sensitivity (e.g., single-photon sensitivity) to enable real-time and/or endpoint monitoring of NA amplification. For example, the first pixel array 152 may be a cooled infrared photodetector based on narrow- or wide bandgap semiconductors, or may be an uncooled photodetector based on pyroelectric and ferroelectric materials, resistive or capacitive microbolometer and/or magnetic based transistors. For example, the second pixel array 154 may include a plurality of avalanche photodiodes (APDs), a quanta image sensor (QIS), a plurality of single-photon avalanche diode (SPAD), and/or the like. The high detection sensitivity of the detector 150 allows the system 100 to lower the detection threshold value relative to the related art, thereby considerably reducing detection/assay time.

As shown in FIG. 1, in some examples, the reaction chamber 130 may be part of a lab-on-a chip with microfluidic inlet, outlet, channels, and components. For example, the reaction chamber may be a well within the lab-on-a-chip. However, embodiments of the present disclosure are not limited thereto, and the reaction chamber 130 may be a test tube, such as an eppendorf tube or a reaction vial, a well plate, or any suitable enclosure.

By using the excitation light to not only excite the NAs but also to heat the solution, the optical system 100 eliminates the need for costly, bulky, and power hungry electronic heating systems and temperature detection hardware. This allows the optical system 100 to be scalable to low-power form factors that may be suitable for IoT (internet-of-things) devices and handheld devices, such as smartphones.

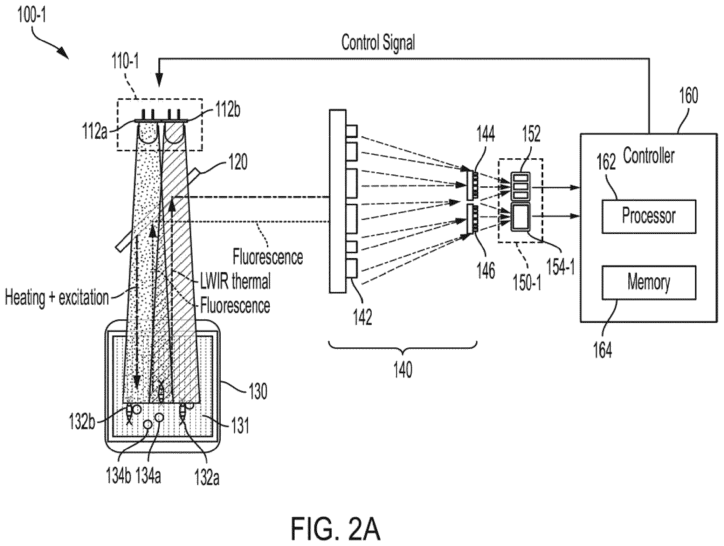

While the optical system 100 is capable of assaying a single type of NA, embodiments of the present disclosure are not limited thereto. FIGS. 2A-2B illustrate examples in which the optical system is capable of assaying two different NAs in the same reaction chamber.

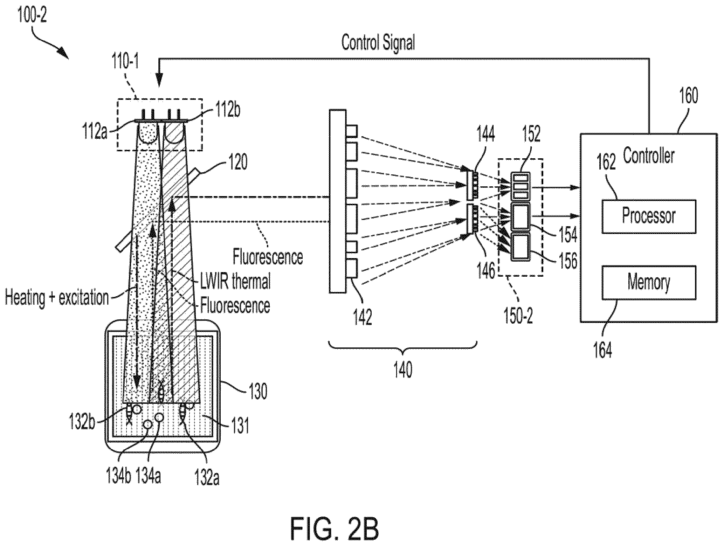

FIGS. 2A-2B are schematic diagrams of compact optical systems 100-1 and 100-2 that are capable of amplification and assay of two different types of NAs in the reaction chamber, according to some exemplary embodiments of the present invention. The optical systems 100-1 and 100-2 are substantially similar to the optical system 100 of FIG. 2, with the exception of modification to certain components that will be described below. For sake of brevity and clarity, only those features of the optical systems 100-1 and 100-2 that are different from the optical system 100 of FIG. 1 may be described herein.

Referring to FIG. 2A, in some embodiments, the solution 131 in the reaction chamber 130 includes more than one type of NA, for example, the first NAs 132a and second NAs 132b, which respond to different excitation lights. Here, the light source 110-1 emits an additional second excitation light toward the reaction chamber 130, which is tuned to excite the second NAs 132b and cause fluorescence of second fluorophores 134b that bind to the second NAs 132b. In some examples, the light source 110-1 includes a green LED 112b that emits the second excitation light as green light in the wavelength range of about 495 nm to about 570 nm. The green LED 112b may be pulsed and have a variable frequency or variable pulse width matching that of the blue LED 112a, as determined by the control signal from the controller 160.

Once the second NAs 132b are excited and amplified by the second excitation light, the solution 131 emits a fourth light that may be a visually orange light having a wavelength in the range of about 560 nm to about 580 nm. The mirror 120 is configured to reflect the fourth light toward the lens module 140.

In some embodiments, the first metalens 142 directs the third and fourth lights toward the third metalens 146, which focuses both of these lights onto the second pixel array 154-1. The second pixel array 154-1 may be capable of hyperspectral imaging in which both the intensity and spectral information of the incoming light may be observed over a wide spectrum of wavelengths, and the controller 160 may be able to monitor and differential the light intensities from the third and fourth lights based on their wavelength ranges. In such embodiments, the controller 160 may utilize the same methodology described above with respect to FIG. 1 to detect and determine the starting concentration of both the first NAs and the second NAs. However, embodiments of the present disclosure is not limited thereto, and as shown in FIG. 2B, the detector 150-1 may include a further pixel array, that is, a third pixel array 156 to detect the fourth light and to generate a second fluorescence light corresponding to the fourth light intensity. In the example of FIG. 2B, the controller 160 (e.g., the processor 162) is further configured to determine the presence of the plurality of second NAs 132b in the solution 131 based on the second fluorescence signal, as described above with respect to FIG. 1.

While FIGS. 2A-2B illustrate examples in which two different NAs are being detected, embodiments of the present disclosure are not limited thereto. For example, using the methodology described above, the optical system may be expanded to detect any suitable number of differing NAs. In some examples, up to 4-6 targets may be detected using fluorescent labels that span in the range of visible to near-infrared.

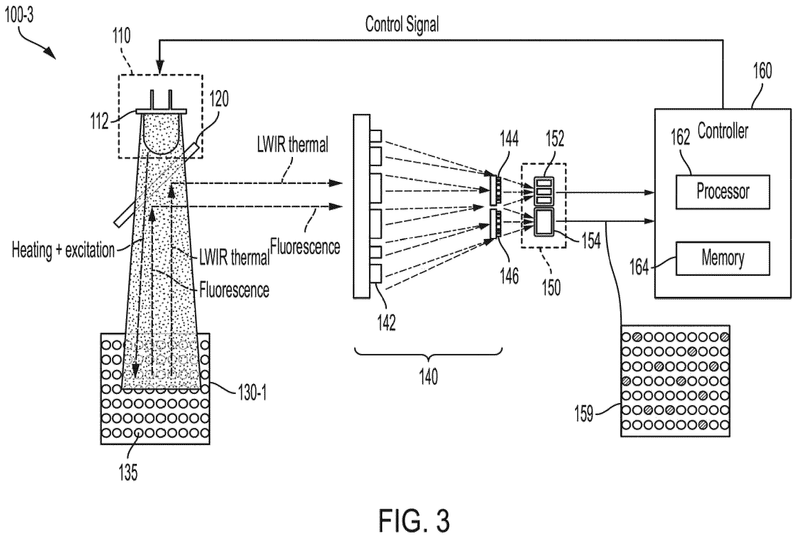

FIG. 3 is a schematic diagram of a compact optical system 100-3 capable of rapid end-point amplification and assay, according to some exemplary embodiments of the present invention.

In some embodiments, the reaction chamber 130 may include a large number of wells 135, each of which may contain a small portion of the solution 131. In some examples, the wells 135 may be only a few micrometers in size and the reaction chamber 130 may include a large number (e.g., tens of thousands) of these wells. The small size of the wells 135 may ensure that each well 135 does not contain more than a small number of first NAs 132 (e.g., less than ten NAs 132). Given that the number of wells 135 may far exceed the number of first NAs 132 and that the distribution of the first NAs across the wells 135 is stochastic/random, many (e.g., most) of the wells 135 may not contain any NAs. Thus, NA amplification may only occur in a subset of the wells, and the remaining wells may not fluoresce.

The controller 160 may perform a sufficient number of thermocycles (e.g., 20-40 cycles) on the reaction chamber 130-1 to ensure that sufficient amplification has occurred to be detectable by the detector 150. Based on the resulting one or more fluorescent lights corresponding to the subset of wells, the second pixel array 154 then produces a two-dimensional pixelated image 159 in which pixels corresponding to the subset of wells are contrasted from the remaining wells that contained no NAs. The controller 160 then counts the number of wells containing NAs 132 in the two-dimensional pixelated image 159 and determines the starting concentration of the first NAs 132 based on a Poisson distribution model.

The rapid-end-point detection performed by the optical system is not limited to a single type of NA, and may be utilized to assay any suitable number of target NAs.

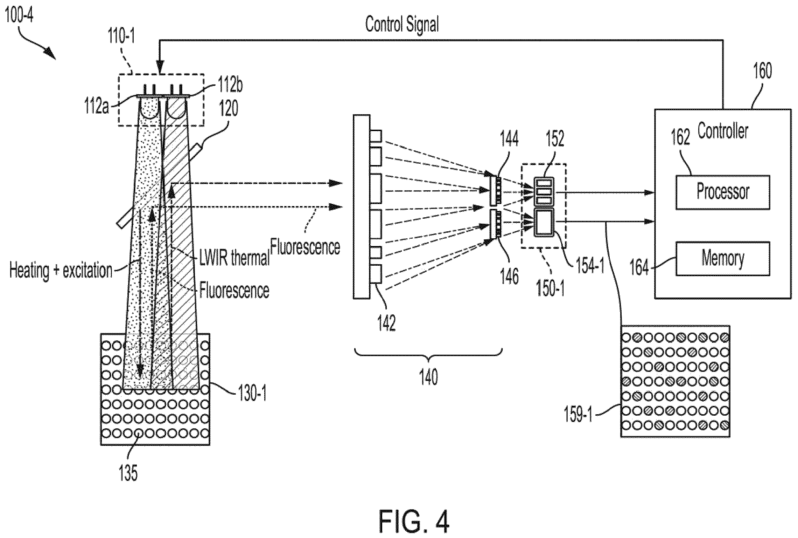

FIG. 4 is a schematic diagram of a compact optical system 100-4 capable of rapid end-point amplification and assay of more than one type of NA, according to some exemplary embodiments of the present invention.

In some embodiments, the optical system 100-4 utilizes the blue and green LEDs 112a and 112b to generate two different excitation lights to excite and amplify the first and second NAs that are present in a subset of the wells 135 within the reaction chamber 130-1. The first and second NAs generate first and second fluorescence lights that can be separately identified via the two-dimensional pixelated image 159-1 generated by the second pixel array 154-1. The controller 160 then counts the number of wells containing each of the first and second NAs and using the Poisson distribution model determines the concentration of each of the two NAs in the reaction chamber 130-1.

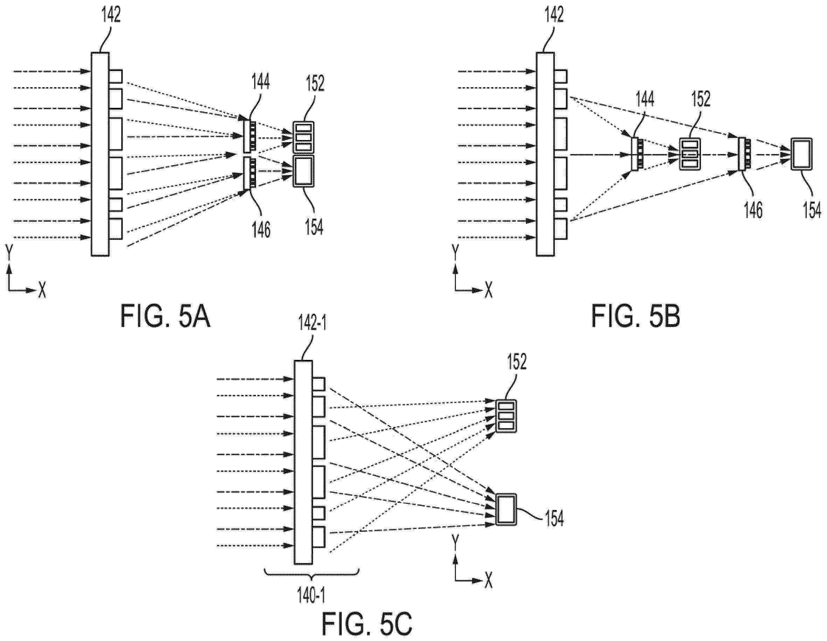

FIGS. 5A-5C are schematic diagrams of the lens module and detector utilizing different focusing lens and pixel array arrangements, according to some embodiments of the present disclosure.

Referring to FIG. 5A, in some examples, the focal lengths of the first metalens for the second and third lights may be the same or substantially the same, and the second and third metalenses 144 and 146 may be offset from one another in a direction (e.g., the Y-direction) crossing (e.g., perpendicular to) an optical axis (e.g., along the X-direction) of the first metalens 142.

Referring to FIG. 5B, in some examples, the focal lengths of the first metalens for the second and third lights may be the different from one another, and the second and third metalenses 144 and 146 may be aligned with one another along an optical axis of the first metalens (e.g., along the X-direction). In this stacked architecture, the second and third metalenses 144 and 146 and the first and second pixel arrays 152 and 156 may be alternately positioned and aligned along the optical axis (along the X-direction) of the first metalens 142. For example, the first pixel array 152 may be positioned between the second and third metalenses 144 and 146, and the third metalens 146 may be positioned between the first and second pixel arrays 152 and 154. In such an example, the second metalens 144 may be transparent to the third light.

Referring to FIG. 5C, the lens module 140-1 includes only a single metalens (142-1) that is configured to directly focus the second and third lights onto the first and second pixel arrays 152 and 154, respectively, without the aid of the any other metalenses. In such examples, the single metalens 142-1 may have a different focal point for each of the second and third lights.

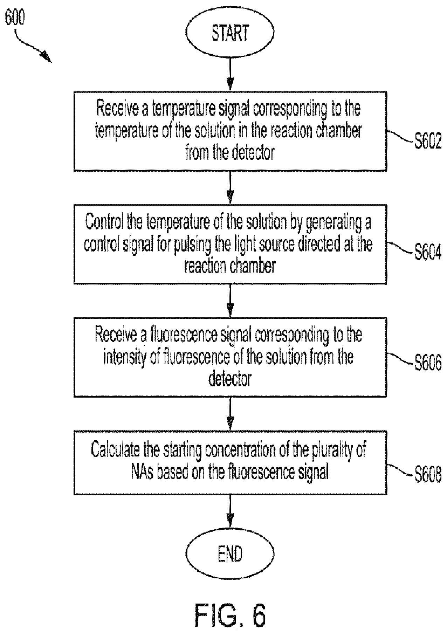

FIG. 6 is a flow diagram illustrating the process 600 of optical NA amplification, according to some embodiments of the present disclosure.

In some embodiments, the controller 160 receives a temperature signal corresponding to a temperature of the solution 131, which includes a plurality of NAs, in a reaction chamber 130 from a detector 150 (S602). The controller 160 then controls the temperature of the solution 131 by generating a control signal for pulsing the light source 110 directed at the reaction chamber 130 (S604). The light source is configured to emit an excitation light toward the solution in response to the control signal, which may have a variable pulse width and be based on the temperature signal and the target temperature (e.g., desired temperature) of the solution. The solution is configured to emit an infrared light in response to heating by the excitation light, and is configured to emit a fluorescence light in response to amplification of the plurality of NAs. The detector generates the temperature signal in response to receiving the infrared light, and generate a fluorescence signal in response to receiving the fluorescence light. The controller 160 receives the fluorescence signal corresponding to the intensity of fluorescence of the solution (S606), and calculates the starting concentration of the plurality of NAs 132 based on the fluorescence signal (S608).

Accordingly, embodiments of the present invention provide a compact optical system that is compatible with all types of qualitative, semi-quantitative, and quantitative NA amplification methods relying on optical detection. By using a detector with single-photon sensitivity such as QIS/APD/SPAD for NA amplification the optical system can considerably reduce the number of cycles and time needed to perform detection/assay. In some embodiments, the optical system is scalable to low-power form factors that are suitable for IoT (internet-of-things) devices and smartphones. The optical system may be adaptable to lab-on-a-chip assays, tubes, well-plates, and all other platforms on which NA amplification may be performed. The optical system is also suitable for both real-time and end-point measurements.

It will be understood that, although the terms "first," "second," "third," etc., may be used herein to describe various elements, components, and/or sections, these elements, components, and/or sections should not be limited by these terms. These terms are used to distinguish one element, component, or section from another element, component, or section. Thus, a first element, component, or section discussed above could be termed a second element, component, or section.

It will be understood that the spatially relative terms used herein are intended to encompass different orientations of the device in use or in operation, in addition to the orientation depicted in the figures. The device may be otherwise oriented (e.g., rotated 90 degrees or at other orientations) and the spatially relative descriptors used herein should be interpreted accordingly. In addition, it will also be understood that when a layer is referred to as being "between" two layers, it can be the only layer between the two layers, or one or more intervening layers may also be present.

The terminology used herein is for the purpose of describing particular embodiments and is not intended to be limiting of the invention. As used herein, the singular forms "a" and "an" are intended to include the plural forms as well, unless the context clearly indicates otherwise. It will be further understood that the terms "include," "including," "comprises," and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Further, the use of "may" when describing embodiments of the invention refers to "one or more embodiments of the invention." Also, the term "exemplary" is intended to refer to an example or illustration.

It will be understood that when an element or component is referred to as being "connected to" or "coupled to" another element or component, it can be directly connected to or coupled to the other element or component, or one or more intervening elements or components may be present. When an element or layer is referred to as being "directly connected to" or "directly coupled to" another element or component, there are no intervening elements or components present.

As used herein, the terms "substantially," "about," and similar terms are used as terms of approximation and not as terms of degree, and are intended to account for the inherent variations in measured or calculated values that would be recognized by those of ordinary skill in the art.

As used herein, the terms "use," "using," and "used" may be considered synonymous with the terms "utilize," "utilizing," and "utilized," respectively.

Also, any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited herein.

The controller 160 and/or any other relevant devices or components according to embodiments of the present invention described herein may be implemented by utilizing any suitable hardware, firmware (e.g., an application-specific integrated circuit), software, or a suitable combination of software, firmware, and hardware. For example, the various components of controller 160 may be formed on one integrated circuit (IC) chip or on separate IC chips. Further, the various components of the controller 160 may be implemented on a flexible printed circuit film, a tape carrier package (TCP), a printed circuit board (PCB), or formed on the same substrate. Further, the various components of the controller 160 may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random access memory (RAM). The computer program instructions may also be stored in other non-transitory computer-readable media such as, for example, a CD-ROM, flash drive, or the like. Also, a person of skill in the art should recognize that the functionality of various computing devices may be combined or integrated into a single computing device, or the functionality of a particular computing device may be distributed across one or more other computing devices without departing from the scope of the exemplary embodiments of the present invention.

## Claims

1. A system (100) for nucleic acid, NA, amplification, the system (100) comprising:
a light source (110) configured to emit a first excitation light based on a control signal;
a reaction chamber (130) configured to house a solution (131) comprising a plurality of first nucleic acids, NAs (132, 132a), the plurality of first NAs (132, 132a) being configured to amplify in response to the first excitation light, the solution (131) being configured to emit a second light in response to heating by the first excitation light and to emit a third light in response to amplification of the plurality of first NAs (132, 132a);
a detector (150) configured to detect the second and third lights and to generate a temperature signal corresponding to the second light and a first fluorescence signal corresponding to the third light;
a lens module (140) configured to focus the second and third lights onto the detector (150); and
a mirror (120) configured to pass-through the first excitation light and to direct the second and third lights toward the lens module (140).

2. The system (100) of claim 1, further comprising:
a controller (160) configured to generate the control signal to pulse the first excitation light based on the temperature signal, the control signal having a variable pulse width and being based on the temperature signal and a desired temperature of the solution (131),
wherein the controller (160) is further configured to determine presence of the plurality of first NAs (132, 132a) in the solution (131) based on the first fluorescence signal.

3. The system (100) of any one of the previous claims, wherein the light source (110) comprises a blue light emitting diode, LED,
wherein the first excitation light has a blue range of wavelengths,
wherein the second light is in a long wavelength infrared, LWIR, range, and
wherein the third light has an orange range of wavelengths.

4. The system (100) of any one of the previous claims, wherein the detector (150) comprises:
a first pixel array (152) configured to detect the second light and to generate the temperature signal corresponding to the second light; and
a second pixel array (154) configured to detect the third light and to generate the first fluorescence signal corresponding to the third light.

5. The system (100) of claim 4, wherein the temperature signal is an average of intensities of light detected by each one of pixels across the first pixel array (152), and
wherein the first fluorescence signal is an average of intensities of light detected by each one of pixels across the second pixel array (154).

6. The system (100) of claim 4 or 5, wherein the second pixel array (154) comprises a cooled infrared photodetector or an uncooled photodetector, and
wherein the second pixel array (154) comprises at least one of an avalanche photodiode, APD, a quanta image sensor, QIS, and a single-photon avalanche diode, SPAD.

7. The system (100) of any one of previous any one of claims 4 to 6, wherein the lens module (140) comprises:
a first metalens (142);
a second metalens (144); and
a third metalens (146),
wherein the first metalens (142) is configured to focus the second light onto the second metalens (144) and to focus the third light onto the third metalens (146), and
wherein the second metalens (144) is configured to focus the second light onto the first pixel array (152), and the third metalens (146) is configured to focus the third light onto the second pixel array (154).

8. The system (100) of claim 7, wherein the second and third metalenses (146) are offset from one another in a direction crossing an optical axis of the first metalens (142).

9. The system (100) of claim 7, wherein the second and third metalenses (146) are aligned with one another along an optical axis of the first metalens (142).

10. The system (100) of any one of the previous claims, wherein the plurality of first NAs (132, 132a) comprises at least one of first RNAs and first DNAs, and
wherein the solution (131) comprises fluorophores that combine with the plurality of first NAs (132, 132a) and fluoresce in response to receiving the first excitation light.

11. The system (100) of any one of the previous claims, wherein the light source (110) comprises a green LED configured to generate a second excitation light in response to the control signal, and
wherein the solution (131) further comprises a plurality of second NAs (132b) being configured to amplify in response to the second excitation light, the solution (131) being configured to emit a fourth light in response to amplification of the plurality of second NAs (132b).

12. The system (100) of claim 11, further comprising:
a third pixel array (156) configured to detect the fourth light and to generate a second fluorescence signal corresponding to the third light; and
a controller (160) configured to determine a concentration of the plurality of second NAs (132b) in the solution (131) based on the second fluorescence signal.

13. The system (100) of any one of the previous claims, wherein the reaction chamber (130) comprises a plurality of wells (135), one or more of the plurality of wells (135) comprising the plurality of first NAs (132, 132a),
wherein the second light comprises one or more fluorescent lights corresponding to the one or more of the plurality of wells (135), and
wherein the first fluorescence signal comprises a two-dimensional image contrasting the one or more of the plurality of wells (135) from other wells (135) of the plurality of wells (135).

14. The system (100) of claim 13, further comprising:
a controller (160) configured to determine a concentration of the plurality of first NAs (132, 132a) in the plurality of wells (135) based on the two-dimensional image.

## Patentansprüche

1. Ein System (100) zur Nukleinsäure, NA, Amplifikation, wobei das System (100) aufweist:
eine Lichtquelle (110), die konfiguriert ist, dass sie basierend auf einem Steuersignal ein erstes Anregungslicht emittiert;
eine Reaktionskammer (130), die konfiguriert ist, dass sie eine Lösung (131) aufnimmt, die eine Mehrzahl von ersten Nukleinsäuren, NAs (132, 132a) aufweist, wobei die Mehrzahl von ersten NAs (132, 132a) konfiguriert ist, dass sie als Antwort auf das erste Anregungslicht amplifiziert, wobei die Lösung (131) konfiguriert ist, dass sie ein zweites Licht als Antwort auf Erwärmen durch das erste Anregungslicht emittiert und ein drittes Licht als Antwort auf Amplifikation der Mehrzahl von ersten NAs (132, 132a) emittiert;
einen Detektor (150), der konfiguriert ist, dass er das zweite und dritte Licht erfasst und ein dem zweiten Licht entsprechendes Temperatursignal und ein dem dritten Licht entsprechendes erstes Fluoreszenzsignal erzeugt;
ein Linsenmodul (140), das konfiguriert ist, dass es das zweite und dritte Licht auf den Detektor (150) fokussiert; und
einen Spiegel (120), der konfiguriert ist, dass er das erste Anregungslicht durchlässt und das zweite und dritte Licht auf das Linsenmodul (140) richtet.

2. System (100) nach Anspruch 1, ferner aufweisend:
eine Steuerung (160), die konfiguriert ist, dass sie das Steuersignal erzeugt, um das erste Anregungslicht basierend auf dem Temperatursignal zu pulsieren, wobei das Steuersignal eine variable Pulsbreite hat und auf dem Temperatursignal und einer SollTemperatur der Lösung (131) basiert,
wobei die Steuerung (160) ferner konfiguriert ist, basierend auf dem ersten Fluoreszenzsignal Vorhandensein der Mehrzahl der ersten NAs (132, 132a) in der Lösung (131) zu bestimmen.

3. System (100) nach einem der vorhergehenden Ansprüche, wobei die Lichtquelle (110) eine blaue Leuchtdiode (LED) aufweist,
wobei das erste Anregungslicht einen blauen Wellenlängenbereich hat,
wobei das zweite Licht in einem langwelligen Infrarot, LWIR, -bereich liegt, und
wobei das dritte Licht einen orangen Wellenlängenbereich hat.

4. System (100) nach einem der vorhergehenden Ansprüche, wobei der Detektor (150) aufweist:
ein erstes Pixelarray (152), das konfiguriert ist, dass es das zweite Licht erfasst und das dem zweiten Licht entsprechende Temperatursignal erzeugt; und
ein zweites Pixelarray (154), das konfiguriert ist, dass es das dritte Licht erfasst und das dem dritten Licht entsprechende erste Fluoreszenzsignal erzeugt.

5. System (100) nach Anspruch 4, wobei das Temperatursignal ein Mittelwert von Intensitäten des Lichts ist, das durch jedes Pixel über das erste Pixelarray (152) erfasst wird, und
wobei das erste Fluoreszenzsignal ein Mittelwert der Intensitäten des Lichts ist, das durch jedes der Pixel über das zweite Pixelarray (154) erfasst wird.

6. System (100) nach Anspruch 4 oder 5, wobei das zweite Pixelarray (154) einen gekühlten Infrarot-Photodetektor oder einen ungekühlten Photodetektor aufweist, und
wobei das zweite Pixelarray (154) mindestens eine Avalanche-Photodiode (APD), einen Quantenbildsensor (QIS) und eine Einzelphotonen-Avalanche-Diode (SPAD) aufweist.

7. System (100) nach einem der vorhergehenden Ansprüche 4 bis 6, wobei das Linsenmodul (140) folgendes aufweist:
eine erste Metalinse (142);
eine zweite Metalinse (144); und
eine dritte Metalinse (146),
wobei die erste Metalinse (142) konfiguriert ist, dass sie das zweite Licht auf die zweite Metalinse (144) fokussiert und das dritte Licht auf die dritte Metalinse (146) fokussiert, und
wobei die zweite Metalinse (144) konfiguriert ist, dass sie das zweite Licht auf das erste Pixelarray (152) fokussiert, und die dritte Metalinse (146) konfiguriert ist, dass sie das dritte Licht auf das zweite Pixelarray (154) fokussiert.

8. System (100) nach Anspruch 7, wobei die zweite und die dritte Metalinse (146) in einer Richtung, die eine optische Achse der ersten Metalinse (142) kreuzt, voneinander verschoben sind.

9. System (100) nach Anspruch 7, wobei die zweite und die dritte Metalinse (146) entlang einer optischen Achse der ersten Metalinse (142) aufeinander ausgerichtet sind.

10. System (100) nach einem der vorhergehenden Ansprüche, wobei die Mehrzahl der ersten NAs (132, 132a) mindestens eine von ersten RNAs und ersten DNAs aufweist, und
wobei die Lösung (131) Fluorophore aufweist, die sich mit der Mehrzahl der ersten NAs (132, 132a) verbinden und als Antwort auf Empfangen des ersten Anregungslichts fluoreszieren.

11. System (100) nach einem der vorhergehenden Ansprüche, wobei die Lichtquelle (110) eine grüne LED aufweist, die konfiguriert ist, dass sie als Antwort auf das Steuersignal ein zweites Anregungslicht erzeugt, und
wobei die Lösung (131) ferner eine Mehrzahl von zweiten NAs (132b) aufweist, die konfiguriert sind, dass sie sich als Antwort auf das zweite Anregungslicht amplifiziert,
wobei die Lösung (131) konfiguriert ist, dass sie ein viertes Licht als Antwort auf die Amplifikation der Mehrzahl von zweiten NAs (132b) emittiert.

12. System (100) nach Anspruch 11, ferner aufweisend:
ein drittes Pixelarray (156), das konfiguriert ist, dass es das vierte Licht erfasst und ein zweites Fluoreszenzsignal erzeugt, das dem dritten Licht entspricht; und
eine Steuerung (160), die konfiguriert ist, dass sie basierend auf dem zweiten Fluoreszenzsignal eine Konzentration der Mehrzahl der zweiten NAs (132b) in der Lösung (131) bestimmt.

13. System (100) nach einem der vorhergehenden Ansprüche, wobei die Reaktionskammer (130) eine Mehrzahl von Vertiefungen (135) aufweist, wobei eine oder mehrere der Mehrzahl von Vertiefungen (135) die Mehrzahl von ersten NAs (132, 132a) aufweist,
wobei das zweite Licht ein oder mehrere Fluoreszenzlichter aufweist, die dem einen oder den mehreren der Mehrzahl von Vertiefungen (135) entsprechen, und
wobei das erste Fluoreszenzsignal eine zweidimensionale Darstellung aufweist, die die eine oder mehreren der Mehrzahl von Vertiefungen (135) von anderen Vertiefungen (135) der Mehrzahl von Vertiefungen (135) unterscheidet.

14. System (100) nach Anspruch 13, ferner aufweisend:
eine Steuerung (160), die konfiguriert ist, dass sie basierend auf der zweidimensionalen Darstellung eine Konzentration der Mehrzahl der ersten NAs (132, 132a) in der Mehrzahl der Vertiefungen (135) bestimmt.

## Revendications

1. Système (100) pour l'amplification des acides nucléiques, NA, le système (100) comprenant:
une source de lumière (110) configurée pour émettre une première lumière d'excitation sur la base d'un signal de commande;
une chambre de réaction (130) configurée pour contenir une solution (131) comprenant une pluralité de premiers acides nucléiques, NA, (132, 132a), la pluralité de premiers NA (132, 132a) étant configurée pour s'amplifier en réponse à la première lumière d'excitation, la solution (131) étant configurée pour émettre une deuxième lumière en réponse au chauffage par la première lumière d'excitation et pour émettre une troisième lumière en réponse à l'amplification de la pluralité de premiers NA (132, 132a);
un détecteur (150) configuré pour détecter la deuxième et la troisième lumières et pour générer un signal de température correspondant à la deuxième lumière et un premier signal de fluorescence correspondant à la troisième lumière;
un module de lentilles (140) configuré pour focaliser la deuxième et la troisième lumières sur le détecteur (150); et
un miroir (120) configuré pour laisser passer la première lumière d'excitation et pour diriger la deuxième et la troisième lumières vers le module de lentille (140).

2. Système (100) de la revendication 1, comprenant en outre:
un contrôleur (160) configuré pour générer le signal de commande afin de pulser la première lumière d'excitation sur la base du signal de température, le signal de commande ayant une largeur d'impulsion variable et étant basé sur le signal de température et une température désirée de la solution (131),
dans lequel le contrôleur (160) est en outre configuré pour déterminer la présence de la pluralité de premières NA (132, 132a) dans la solution (131) sur la base du premier signal de fluorescence.

3. Système (100) de l'une quelconque des revendications précédentes, dans lequel la source de lumière (110) comprend une diode électroluminescente, DEL, bleue,
dans lequel la première lumière d'excitation est d'une bande spectrale bleue,
dans lequel la deuxième lumière est d'une bande spectrale infrarouge lointain, LWIR, et
dans lequel la troisième lumière est d'une bande spectrale orange.

4. Système (100) de l'une quelconque des revendications précédentes, dans lequel le détecteur (150) comprend:
une première disposition des pixels (152) configurée pour détecter la deuxième lumière et pour générer le signal de température correspondant à la deuxième lumière; et
une deuxième disposition des pixels (154) configurée pour détecter la troisième lumière et pour générer le premier signal de fluorescence correspondant à la troisième lumière.

5. Système (100) de la revendication 4, dans lequel le signal de température est une moyenne des intensités de la lumière détectée par chacun des pixels de la première disposition des pixels (152), et
dans lequel le premier signal de fluorescence est une moyenne des intensités de la lumière détectée par chacun des pixels de la deuxième disposition des pixels (154).

6. Système (100) de la revendication 4 ou 5, dans lequel la deuxième disposition des pixels (154) comprend un photodétecteur infrarouge refroidi ou un photodétecteur non refroidi, et
dans lequel la deuxième disposition des pixels (154) comprend une photodiode à avalanche, APD, et/ou un capteur de type QIS [QIS =quanta image sensor] et/ou une diode à avalanche monophotonique, SPAD.

7. Système (100) de l'une quelconque des revendications précédentes 4 à 6, dans lequel le module de lentilles (140) comprend:
une première métalentille (142);
une deuxième métalentille (144); et
une troisième métalentille (146),
dans lequel la première métalentille(142) est configurée pour focaliser la deuxième lumière sur la deuxième métalentille (144) et pour focaliser la troisième lumière sur la troisième métalentille (146), et
dans lequel le deuxième métalentille (144) est configuré pour focaliser la deuxième lumière sur la première disposition des pixels (152), et la troisième métalentille (146) est configuré pour focaliser la troisième lumière sur la deuxième disposition des pixels (154).

8. Système (100) de la revendication 7, dans lequel la deuxième et la troisième métalentilles (146) sont décalées l'une par rapport à l'autre dans une direction qui croise un axe optique de la première métalentille (142).

9. Système (100) de la revendication 7, dans lequel la deuxième et la troisième métalentilles (146) sont alignées l'une avec l'autre le long d'un axe optique de la première métalentille (142).

10. Système (100) de l'une quelconque des revendications précédentes, dans lequel la pluralité de premières NA (132, 132a) comprend des premiers ARN et/ou des premiers ADN, et
dans lequel la solution (131) comprend des fluorophores qui se combinent avec la pluralité de premières NA (132, 132a) et deviennent fluorescents en réponse à la réception de la première lumière d'excitation.

11. Système (100) de l'une quelconque des revendications précédentes, dans lequel la source de lumière (110) comprend une DEL verte configurée pour générer une deuxième lumière d'excitation en réponse au signal de commande, et
dans lequel la solution (131) comprend en outre une pluralité de deuxième NA (132b) étant configurée pour s'amplifier en réponse à la deuxième lumière d'excitation, la solution (131) étant configurée pour émettre une quatrième lumière en réponse à l'amplification de la pluralité de deuxième NA (132b).

12. Système (100) de la revendication 11, comprenant en outre:
une troisième disposition des pixels (156) configurée pour détecter la quatrième lumière et pour générer un deuxième signal de fluorescence correspondant à la troisième lumière; et
un contrôleur (160) configuré pour déterminer une concentration de la pluralité de secondes NA (132b) dans la solution (131) sur la base du deuxième signal de fluorescence.

13. Système (100) de l'une quelconque des revendications précédentes, dans lequel la chambre de réaction (130) comprend une pluralité de puits (135), un ou plusieurs de la pluralité de puits (135) comprenant la pluralité de premières NA (132, 132a),
dans lequel la deuxième lumière comprend une ou plusieurs lumières fluorescentes correspondant à l'un ou plusieurs de la pluralité de puits (135), et
dans lequel le premier signal de fluorescence comprend une image bidimensionnelle contrastant l'un ou plusieurs des puits (135) de la pluralité de puits (135) par rapport aux autres puits (135) de la pluralité de puits (135).

14. Système (100) de la revendication 13, comprenant en outre:
un contrôleur (160) configuré pour déterminer une concentration de la pluralité de premières NA (132, 132a) dans la pluralité de puits (135) sur la base de l'image bidimensionnelle.
